Europäisches Patentamt

(19) European Patent Office

Office européen des brevets

(11) Publication number: **0 313 318 B1**

(12) **EUROPEAN PATENT SPECIFICATION**

(45) Date of publication of patent specification :
12.06.91 Bulletin 91/24

(51) Int. Cl.⁵ : **G01N 33/555, // G01N33/569**

(21) Application number : **88309773.5**

(22) Date of filing : **19.10.88**

(54) Immunoassay.

(30) Priority : **20.10.87 GB 8724549**

(43) Date of publication of application :
**26.04.89 Bulletin 89/17**

(45) Publication of the grant of the patent :
**12.06.91 Bulletin 91/24**

(84) Designated Contracting States :
**AT BE CH DE ES FR GB GR IT LI LU NL SE**

(56) References cited :
**EP-A- 0 058 780**
**EP-A- 0 132 537**
**EP-A- 0 189 389**
**EP-A- 0 194 156**
**WO-A-83/02009**

(73) Proprietor : **THE WELLCOME FOUNDATION LIMITED**
**Unicorn House 160 Euston Road**
**London NW1 2BP (GB)**

(72) Inventor : **Coombs, Robin Royston Amos**
**6 Selwyn Gardens**
**Cambridge (GB)**

(74) Representative : **Stott, Michael John et al**
**The Wellcome Research Laboratories Group**
**Patents & Agreements Langley Court**
**Beckenham Kent BR3 3BS (GB)**

## Description

This invention relates to immunoassays and to test kits for use in immunoassays. More particularly, it relates to the use of inagglutinable bovine red cells in an immunoassay.

IgM antibody capture assays were first described for the detection of hepatitis A and rubella virus-specific IgM (Duermeyer et al, J. Med. Virol. 4, 25-32, 1979 ; Flehming et al, J. Inf. Dis. 140, 169-175, 1979 ; Krech and Wilhelm, J. Gen. Virol. 44, 281-286, 1979). Since then this technique has been applied to the diagnosis of many microbial infections. Price (J. Immunol. Methods 32 261-273, 1980) and Wreghitt and Sillis (J. Hyg. Camb. 94, 217-227, 1985) developed a μ-capture ELISA for detecting Mycoplasma pneumoniae-specific IgM, and found it to be a sensitive, reliable and specific test.

A new antigen-specific immunoglobulin capture test has now been devised. So-called inagglutinable bovine red cells coupled with an anti-immunoglobulin reagent adsorb the corresponding immunoglobulin when added. They do not haemagglutinate at this stage due to the unusual structure of the cell membrane. However, the bovine red cells will subsequently agglutinate when presented with the corresponding and appropriate antigen. This phenomenon enables an assay both for antibody and for antigen to be provided.

Accordingly the present invention provides a method of assaying a sample for an antibody specific for a predetermined antigen having multiple epitopes or for a predetermined antigen having multiple epitopes, which method comprises :

(i) contacting inagglutinable bovine red blood cells to which an immunoglobulin-binding agent is attached with the sample in the case of assay for the antibody or with antibody specific for the antigen in the case of assay for the antigen ;

(ii) contacting the thus-treated red cells with antigen for which the antibody is specific in the case of assay for the antibody or with the sample in the case of assay for the antigen ; and

(iii) determining whether haemagglutination occurs.

Such an assay has a number of advantages, for example for antigen-specific IgM over the equivalent μ-capture ELISA assay. Benefits are the simplicity of the assay, the time required which is from one to two hours instead of twenty-four hours for the ELISA assay and that no special equipment is needed. Antibody, typically monoclonal antibody, can be used without preliminary purification or direct coupling to the red cells in step (i) in assay for antigen.

In the accompanying drawings :

Figure 1 is a diagrammatic representation of an antigen-specific IgM red cell capture assay according to the invention :

Figure 2 is a comparison of a red cell IgM-antibody capture assay (left) with μ-capture ELISA (right) ;

Figure 3 is a diagrammatic representation of an assay for antigen according to the invention using captured monoclonal antibody ;

Figure 4 shows the correlation between red cell IgM antibody capture titre and μ-capture ELISA units of IgM for 47 sera from patients with clinical features that suggested M. pneumoniae infection (Example 1) ;

Figure 5 shows the amount of M. pneumoniae-specific IgM detected by red cell IgM antibody capture test (▲) and μ-capture ELISA (●) in sera taken at various times after proven M. pneumoniae infection (Example 1), with the dotted line indicating positive cut-off values ;

Figure 6 is a diagrammatic representation of the red-cell IgE-antibody capture assay of Example 3, showing the dilutions at which haemagglutination ocurred ; and

Figure 7 shows the results of the assay for chlamydia antigen in Example 4 using (a) unstabilised "inagglutinable" bovine red cells coupled with anti-mouse IgG or (b) stabilised anti-mouse IgG-coupled bovine red cells. ▨ denotes positive RPH following half hour incubation at room temperature. ■ denotes positive RPH following overnight incubation at room temperature.

The assay requires the use of inagglutinable bovine (bovidae) red blood cells. Unlike the red cells of most animals, bovine red cells are not agglutinated readily by specific antibody. Even when fully sensitised only certain samples can be agglutinated, and then only to a degree far below that which might be expected from the haemolytic titre of the sensitising serum (Coombs et al, Brit. J. exp. Path. 32, 195-202, 1951).

Inagglutinable bovine red blood cells are a recognised type of cells. See too Gleeson-White et al, Brit. J. exp. Path. 31, 321-331, 1950 ; Uhlenbruck et al, Vox Sang. 12, 420-428, 1967 ; and Edebo et al, Scand. J. Immunol. 12, 193-201, 1980. The inagglutinable cells may be arbitrarily sub-divided into three classes on the basis of their relative agglutinability, using the IgM Paul-Bunnell antibody (Gleeson-White et al, 1950). The terms "more agglutinable", "intermediate" and "more inagglutinable" describe the three classes. These classes were originally termed "markedly agglutinable", "moderately agglutinable" and "inagglutinable" by Gleeson-White et al. Even the "more agglutinable" red blood cells are inagglutinable in comparison with other cell types, however. Any of the three classes of cells may therefore be used in the assay.

EP 0 313 318 B1

An antiglobulin or other immunoglobulin-binding agent is attached to the inagglutinable red cells. Any appropriate immunoglobulin-binding agent could be used. The binding agent, however, must not block the antigen-binding site of the immunoglobulin to which it becomes attached. Also, there may be a need for the binding agent to be specific for one of the immunoglobulin classes IgG, IgA, IgM, IgD and IgE. Preferably, antibody assay is conducted for immunoglobulin of a particular isotype.

Anti-$\gamma$ antibody may be used as the immunoglobulin-binding agent where it is wished to bind IgG. Similarly, anti-$\alpha$, anti-$\mu$, anti-$\delta$ and anti-$\epsilon$ antibody may be used to bind IgA, IgM, IgD and IgE respectively. The antibody typically is specific for the species of immunoglobulin to be captured. Other immunoglobulin-binding agents are those capable of binding to the Fc portion of an immunoglobulin, such as protein A or the avidin-biotin system.

Chromic chloride is preferably used to couple the immunoglobulin binding agent to the inagglutinable red cells. Any other appropriate coupling agent may be used, however.

Assay for Antibody (Antibody Capture Assay)

The antibody capture assay is based on the adsorption or "capture" of antibody, for example of a particular isotype, from a sample onto the inagglutinable bovine red cells to which an immunoglobulin-binding agent is attached. No haemagglutination (actually reverse passive haemagglutination or RPH) occurs at this stage because the captured antibody molecule cannot bridge across and bind cells together due to the long mucopeptide chains extending from the protein backbone of the red cell membrane where the immunoglobulin-binding agent is coupled. Only on addition of a further antigenic molecule reacting specifically with the captured antibody does agglutination (or rather RPH) occur. The molecular chain presumably now is thus of a dimension which reaches beyond the "effective periphery" of the red cell membrane and thus is able to bridge cell to cell.

In more detail, the test sample is typically human serum. The sample is generally diluted in a buffer solution. A suspension of the red cells to which an immunoglobulin-binding agent is attached may then be added and incubated. The cells can then be removed, washed and resuspended in buffer solution. Antigen for which the antibody under assay is specific may then be added and RPH determined. The antigen may be presented coupled to a larger moiety, for example IgM or, preferably, agglutinable sheep red blood cells. Such cells may be obtained by treating sheep red blood cells with chymotrypsin. The titre of antibody in a particular sample can easily be titrated by testing serial dilutions of the sample. In the case of a serum sample, a high titre indicates recent infection with the corresponding microorganism.

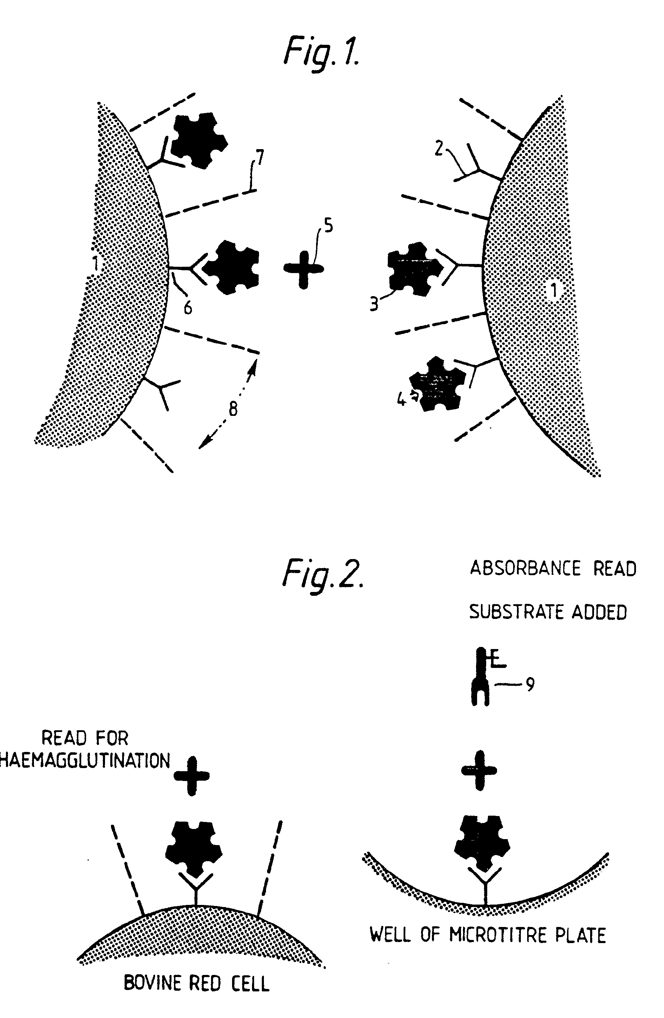

The IgM antibody capture assay is illustrated in Figure 1. Coupled to inagglutinable bovine red cells (1), for example of the intermediate class, is anti-$\mu$ antibody (2). This may be sheep anti-human $\mu$ coupled to the red cells by chromic chloride. On contacting such red cells (1) with patients' serum, IgM (3) is captured. This IgM (3) has antigen-specific combining sites (4). The appropriate antigen (5) such as M. pneumoniae, is added and RPH occurs. There is a protein backbone (6) to the red cell membrane. Muco-peptide chains (7) with attached sialic acid render the cells inagglutinable. However, the molecular chain extends beyonds the effective periphery (8) of the red cell membrane when the antigen (5) is added. Hence RPH takes place.

Such an IgM-antibody capture test is simple to perform and does not require expensive washing or reading equipment. This applies too to the capture assays for other antibodies. The test can be performed in one to two hours in comparison with the $\mu$-capture ELISA which takes 24 hours. Thus, the IgM-antibody capture test is amenable to rapid diagnosis, which is important since M. pneumoniae infection responds to antibiotic therapy.

The IgM-antibody capture assay is compared with the $\mu$-capture ELISA assay in Figure 2. The symbols are as in Figure 1. The IgM-antibody capture assay (left) involves capture of IgM from a sample followed by addition of antigen. Haemagglutination (RPH) can then be read. On the other hand, the $\mu$-capture ELISA (right) involves many more stages : capture of IgM from a sample, addition of antigen, addition of an enzyme-linked antibody (9) capable of binding to the antigen, addition of enzyme substrate. Finally, the absorbance is read. There are intermediate washing steps to be taken into account too.

Antigen Assay

The principles on which the antibody capture assay is based can also be used for antigen assay. Antibody specific for the antigen, it is wished to assay is brought into contact with, and binds to, the red cells to which the immunoglobulin binding agent is attached. This antibody may be polyclonal antibody but is preferably monoclonal antibody. Direct coupling of the antibody onto the red cells is thereby avoided. Very small amounts of the antibody can be used. Relatively impure antibody preparations may be used such as ascitic fluids or culture supernates. Dialysis of a monoclonal antibody preparation, for example, is therefore unnecessary.

A test sample is then brought into contact with the red blood cells to which the antibody is linked through the immunoglobulin-binding agent. The added test sample may be human serum or a secretion or excretion

3

product. Antigen in the sample which is specific for the antibody binds to the antibody. RPH occurs and may be monitored. As the assay depends upon the antibody specific for the antigen under assay being captured onto the anti-immunoglobulin coupled to the inagglutinable red cells, it may be termed "Capture RPH".

In practice, a suspension of the red cells to which an appropriate immunoglobulin binding agent is linked and a solution of antibody specific for the antigen under assay are mixed and incubated. The cells may then be removed, washed and resuspended in buffer solution. The test sample, typically diluted with a buffer, is then added and RPH monitored.

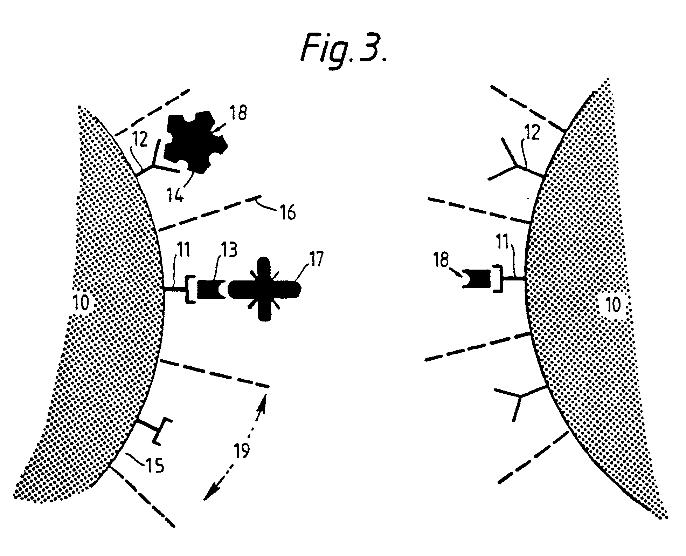

The procedure involved in a capture RPH is illustrated in Figure 3. Coupled onto the inagglutinable red cells (10), for example of the intermediate class, are immunoglobulin-binding antibodies. In the present instance, these are anti-γ antibody (11) and anti-μ antibody (12). Both antibodies may be sheep anti-mouse immunoglobulin coupled to the red cells (6) by chromic chloride. The red cells (10) are then brought into contact with monoclonal antibody specific for the antigen under assay. IgG monoclonal antibody (13) and IgM monoclonal antibody (14) are captured by the anti-γ antibody (11) and anti-μ antibody (12) respectively. The monoclonal antibodies will be of mouse origin when the antiglobulins are anti-mouse immunoglobulin.

Extending from the protein backbone (15) of the red cell membrane are muco-peptide chains (16) with attached silaic acid. These chains render the cells relatively inagglutinable. On addition of a microbial antigen (17) with repeating epitopes recognised by the antigen-combining sites (18) of the monoclonal antibodies, however, the molecular chain extends beyond the effective periphery (19) of the red cell membrane. RPH may thus occur.

Antigen can be detected in samples of large volumes. Such a sample may be incubated with a suspension of antibody-linked red cells for, for example, from 30 minutes to 24 hours at room temperature. The samples may be from 100 μl to 10 ml or even more in volume, although for the larger samples a longer incubation is necessary. Affinity absorption of the antigen onto the antibody-carrying red cells occurs.

The red cells with their affinity-captured antigen are then centrifuged down and resuspended in a much smaller volume of liquid, such as their own supernatant fluid. RPH can be read. This assay procedure can be particularly useful in tests on urine and faecal extracts, as well as other body fluids where large volumes may be available for testing.

The components required for assay of an antibody specific for a predetermined antigen having multiple epitopes or for a predetermined antigen having multiple epitopes may be provided in a test kit. Such a kit comprises :

(i) inagglutinable bovine red blood cells to which an immunoglobulin-binding agent is attached ; and
(ii) in the case of assay for antibody, antigen for which the antibody is specific or, in the case of assay for antigen, antibody specific for the antigen.

Preferably, component (i) is provided in stabilised form according to GB-A-2138132. The red blood cells with the immunoglobulin-binding agent attached are treated with a α, ω-aliphatic dialdehyde, generally glutaraldehyde, and are freeze-dried.

The test kit may include other components such as a buffer solution, a wash solution and/or a control solution of the antibody being assayed for or of the antigen being assayed for, as the case may be.

The assay may be conducted to achieve qualitative or semi-quantitative results. Any antigen with more than one epitope, and any antibody to such an antigen, may be assayed for. Multiple epitope antigens are essential in step (ii) of the present assay to cause RPH. Preferably, the epitopes are identical. Assays may therefore be conducted for microbial antigens, for example bacterial, viral or fungal, and antibodies against such antigens.

The assay should be conducted in the knowledge that haemagglutination will occur when the antibody being assayed for is present in the sample under test in the case of assay for the antibody or when the antigen being assayed for is present in the sample under test in the case of assay for the antigen. The antibody which binds to the immunoglobulin-binding agent in step (i) and the corresponding antigen which binds to this antibody in step (ii) need to be together sufficiently large such that RPH occurs in step (iii). When IgM is the antibody in step (i) or a microbial antigen is the antigen in step (ii), this will not be a problem.

A control assay should be run before or alongside an assay according to the invention. In the control assay, steps (i) to (iii) are followed except that a control solution of the antibody being assayed for or of the antigen being assayed for replaces the sample under test. RPH will occur in step (iii) when the antibody which binds to the immunoglobulin-binding agent in step (i) and the corresponding antigen which binds thereto in step (ii) are big enough. Typically, a moiety of 200 kD or more which comprises the antigen is used. The antigen may be coupled to IgM or, better, agglutinable sheep red blood cells to achieve this threshold if necessary.

Tests can be effected to reveal infections attributable to a range of microbial pathogens causing human disease. For example, antibody for M. pneumoniae and other human pathogens such as Chlamydia psittaci and Brucella abortus may be detected. Raised IgM antibody indicates present or recent infection. Direct antigen

4

assays may be effected for, for example, Chlamydia psittaci, rotavirus, herpes simplex virus type I or II or meningococcal polysaccharide A or B.

The following Examples illustrate the invention :

## Example 1. Detection of M. pneumoniae-specific IgM.

### Serum samples

A total of 50 samples of serum from 41 patients with clinical features that suggested M. pneumoniae infection were selected. All the samples had M. pneumoniae complement fixation (CF) antibody titres of $\geqslant$ 64 or were corresponding acute phase sera with lower titres. A serum with M. pneumoniae CF titre $\geqslant$ 256 and with the highest M. pneumoniae-specific IgM titre was chosen as the reference positive control serum in the μ-capture ELISA. This was assigned an arbitary value of 100 units of M. pneumoniae-specific IgM per 0.1 ml. This positive control serum was diluted in negative control serum to give dilutions containing 33, 10, 3.3, 1.0, 0.33 and 0.1 units.

Thirty samples of serum from 10 patients taken at intervals of up to nine months after culture-proven M. pneumoniae infection were also studied to see how long after infection the specific IgM could be detected.

### μ-capture ELISA

This was performed as described by Wreghitt and Sillis (1985). Sera with $\geqslant$ 0.33 arbitary units of M. pneumoniae-specific IgM per 0.1 ml were regarded as positive.

### Mycoplasma pneumoniae antigen

This was prepared as described by Wreghitt and Sillis (1985). Briefly, the FH strain of M. pneumoniae was grown in 8 ml of modified Hayflick's broth with 1% glucose and phenol red as indicator (Leach, J. Gen. Microbiol. 75, 135-153, 1973). The culture was grown to form an adherent monolayer in a 113 g (4 oz) 'medical flat' on its side during incubation at 37°C until an acid colour developed. The spent broth was regularly replaced and the bottle reincubated until the monolayer was confluent, when the broth was decanted, and the monolayer gently washed with phosphate-buffered saline (PBS) pH 7.6. The antigen was then scraped from the glass, resuspended in PBS, and then frozen and thawed once, sonicated at maximum setting for 10 min, and stored in 1 ml aliquots at –20°C until required.

### Selection of the inagglutinable bovine red cells

For the present tests – see Figure 1 – the appropriate red cell is the more agglutinable red cell of the bovine species – which displays red cells of varying degrees of inagglutinability. The red cell was selected by being relatively agglutinable with the Paul-Bunnell antibody both on direct agglutination and in an antiglobulin test (Edebo et al, Scand. J. Immunol. 12, 193-201, 1980).

### Anti-human μ-Fc reagents

These antisera were raised in sheep and, as IgG-rich fractions, were rendered μ-specific and tested for isotype-specificity as described by Coombs et al. (Immunology 33, 1037-1044, 1978). The antibodies were designated Z368F and Z631V.

### Coupling bovine red cells with polyclonal sheep anti-μ reagents

The anti-μ reagents were coupled to bovine red cells, without previous enzyme-treatment, with the chromic chloride procedure (Siddle et al, J. Immunol. Methods 73, 169-176, 1984). The effectiveness of the coupling procedure was checked by making a similar coupling to enzyme-treated sheep red cells. The coupling onto bovine red cells is not so easily checked because of the relative inagglutinability of the red cells. Coupling onto bovine red cells can be checked by performing a capture test as described.

The specificity of the two IgM capturing antibodies Z368F and Z631V was established by coupling them onto chymotrypsin-treated sheep red cells and using these in reverse passive haemagglutination tests against two monoclonal human IgM anti-rhesus antibodies and two IgG monoclonal antibodies. They reacted only with the human IgM monoclonal antibodies.

Performance of the red cell IgM antibody capture assay

Human sera were titrated from a 1 in 10 dilution with 10 or 4 fold dilution steps, the last tube having PBS diluent alone. Bovine red cells coupled with anti-human μ were added as a 1% suspension in equal volume. After 15 min. incubation at room temperature the cells were deposited by light centrifugation, given one wash and resuspended to a 1% suspension. The washed cells (30 μl) were set out in 2 rows in a microtitre plate. To one row was added 30 μl of a 1 in 10 dilution of the M. pneumoniae antigen and to the other 30 μl of PBS diluent.

The plates were shaken and read for haemagglutination (RPH) when the red cells settled.

Stabilisation and freeze drying of the anti-μ coupled bovine red cells

Stabilisation was performed with a 0.01% solution of glutaraldehyde as described by Cranage et al (J. Immunol. Methods 64, 7-16, 1983). The mixture for freeze-drying was PBS containing 1.5% sucrose (w/v) and 1% bovine plasma albumin (batches first tested for suitability).

RESULTS

Preliminary investigations on the antigen-specific IgM red cell capture assay

Two polyclonal μ-specific sheep IgG anti-human IgM reagents (Z631 and Z368) were used in these preliminary experiments. The IgM specificity of these reagents was shown in tests reported by Coombs et al, 1978. Coupling these Ig reagents at 2.0, 1.0 or 0.5 mg/ml with chromic chloride to chymotrypsin-treated sheep red cells gave a positive RPH reaction with a 1 in 40,000 dilution of human serum – measuring its IgM content.

Similar coupling (at 2.0 mg/ml) to non-enzyme-treated bovine red cells (E6-in the more agglutinable class-Edebo et al. 1980) resulted in coupled red cells which did not agglutinate (RPH) when added to serial dilutions of human serum. However, the IgM in the serum was adsorbed on to the bovine red cells. This could be shown by washing the red cells with adsorbed IgM and mixing with enzyme-treated sheep red cells coupled with the same anti-IgM reagent, in a sort of mixed 2-stage RPH. With the anti-globulin linked E6 bovine red cells, IgM captured from human serum could (after washing) be shown to a titre of 5,000, which (see above) was a lower titre than found in the usual homogeneous RPH using the same antibody coupled to enzyme-treated sheep red cells.

The next experiment showed that in the acute stage of M. pneumoniae infection, some of the sera IgM which could be captured by anti-IgM coupled E6 bovine red cells had antigen (i.e. M. pneumoniae) specificity. This was shown by adding an M. pneumoniae antigen preparation to the cell suspensions with captured IgM. In performing a test, anti-μ coupled E6 bovine red cells were added to dilutions of patients' sera – from 1 in 10 in a 10 fold dilution series. After a short incubation, the red cells were centrifuged, washed once in PBS and then added as a 1% suspension to the M. pneumoniae antigen. Strong haemagglutination was seen up to a 1 in 10,000 dilution of the serum. The optimal M. pneumoniae antigen dilution was 1 in 10 ; but agglutination of varying strength was found up to a dilution of 1 in 1,000. No agglutination was found in the absence of antigen.

Table 1 records the results of red cell IgM antibody capture tests with known positive and negative sera and six sera tested blind. Results using the μ-capture ELISA are also recorded and show good agreement. Also shown are results of assays of total IgM in the eight sera using both the mixed 2-stage RPH described above and the usual homogeneous RPH assay. It is clear that the antigen-specific IgM detected is not related to the assay for total IgM (non-antigen-specific).

Comparative tests using the red cell IgM-capture system and ELISA μ-capture Assay

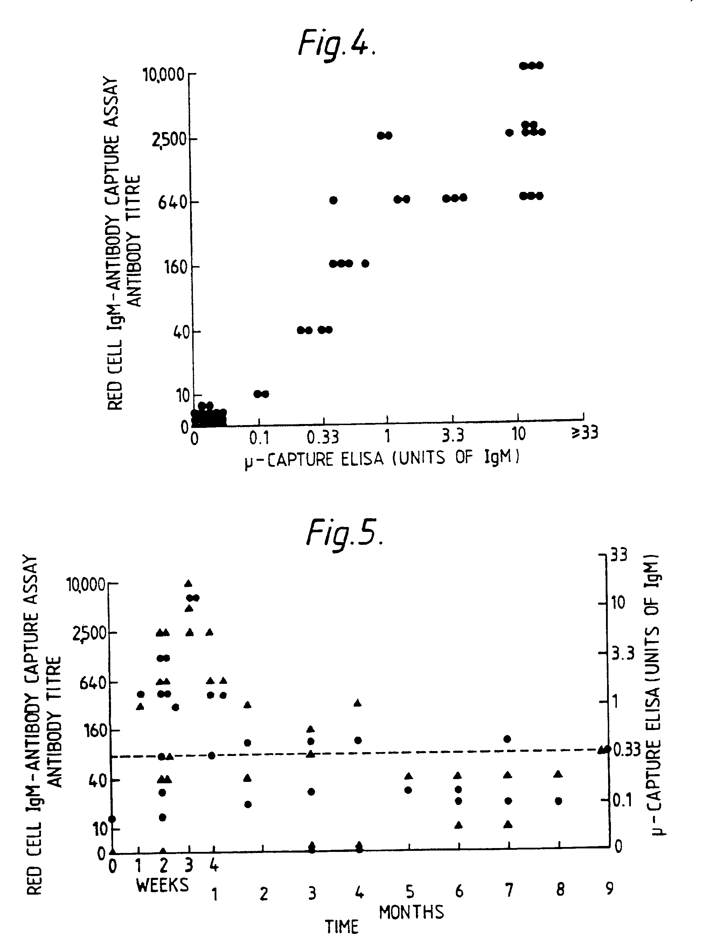

Stored sera previously tested by the μ-capture ELISA were tested by the red cell IgM antibody capture-assay at dilutions of 1 in 40, 1 in 160, 1 in 640, 1 in 2500 and 1 in 10000. The results obtained with the two test procedures are shown in Figure 4, where it can be seen that there is good agreement between the results of the two assays, and that the two tests have similar sensitivity.

There were three serum samples taken from one patient at various times after M. pneumoniae infection, which agglutinated the control cells to a titre of 640 (where PBS was used instead of antigen). These sera were excluded from Fig 4 because their true titre could not be established.

The results of μ-capture ELISA and red cell IgM antibody capture tests on sera taken at various times after proven M. pneumoniae infection are shown in Figure 5. It can be seen that M. pneumoniae-specific IgM was detected for a similar time after infection in both assays (approximately 4-6 months).

Capturing antibodies coupled to the red cells were specific for human IgM. As would be expected it could be shown that prior treatment of the patient's serum under test with dithiothreitol (DTT) – a disulphide bond reducing agent – ablated the reaction. The sera were treated with either 30 mM, 20 mM or 10 mM DTT at 37°C for 40 min. Even the 10 mM concentration removed all activity. Similar treatment of an IgG monoclonal antibody to Chlamydia psittaci did not remove its demonstration in a corresponding antigen-specific IgG red cell capture assay.

Example 2 : Detection of Chlamydia psittaci, rotavirus, herpes simplex viruses types I and II and meningococcal polysaccharides A and B

Selection of Red Cells

The bovine red cells (Ox E6) were used. Though relatively inagglutinable when compared to other species, they were classified as moderately agglutinable bovine cells by their sensitivity to agglutination by Paul Bunnell antibody (in direct agglutination) and by antiglobulin test (Gleeson-White et al, Brit. J. Exp. Pathol. 31, 321-331, 1950). The red cells were deposited by centrifugation (3000 rpm, MSE Centaur 2) and buffy coat and serum removed. The red cells were then washed five times in 0.15 M NaCl before coupling.

Selection of Antibodies and Antigens Capturing Antibodies :

A sheep anti-mouse immunoglobulin designated A366 was used. This was purified by ammonium sulphate precipitation followed by ion exchange chromatography on DE52 cellulose (Whatman). A sheep anti-mouse γ Fc reagent designates AX609C and a sheep anti-mouse μ Fc reagent designated AX706E were also employed.

Monoclonal antibodies to microbial agents

i. Anti-Chlamydia psittaci

Monoclonal antibodies designated CF6/J12 (IgG2a), CF2/47 (IgG3), CF6/L13 (IgG2a), CF4/B2 (IgG3) and CF6/L5 (IgG2a) were raised as described in Thornley et al (J. Gen. Microbiol., 131, 7-15, 1985).

ii. Anti-rotavirus

Mouse monoclonal A3M4 (IgG2a) was used. It was prepared from ascites by 40% ammonium sulphate precipitation and its use in RPH assays has been described (Cranage et al, J. Virol. Methods 11, 273-287, 1985).

iii. Anti-Herpes simplex virus

Four mouse monoclonals were employed. These were against the type common glycoprotein D (AP7 and LP2), the major DNA binding protein (Buckmaster et al J. Med. Virol. 13, 193-202, 1984) and the type 1 glycoprotein G [(LP10), Richman et al, J. Virol. 57, 647-655, 1986]

iv. Anti-meningococcal polysaccharide A and B

Mouse monoclonal antibodies WMA/33, (IgG$_2$b) raised against meningococcal polysaccharide A and MB67/A7, (IgM) monoclonal against meningococcal polysaccharide B were employed.

Microbial Antigens Chlamydia psittaci : Psittacosis CF antigen (batch no 8202/1) was obtained from the Public Health Laboratory Service (London).

Rotavirus : A 10% faecal extract from an infected infant was prepared (Cranage et al, 1985) and preserved as such in PBS at −12°C.

Herpes simplex viruses types I and II : Antigens employed w ere sonicates of strain HFEM (Type I) and HVD (25766) (Type II) and stored frozen.

Meningococcal polysaccharides A and B : These were provided as solutions (1 mg/ml) and used at the dilutions shown in the text.

Coupling procedure :

All immunoglobulins were dialysed against 0.15 M NaCl before use and were coupled at an optimal concentration of 1-2 mg/ml to equal volumes of packed red blood cells using chromic chloride as described in Example 1.

Procedure for performing Capture RPH

Red cells coupled with the anti-mouse immunoglobulin reagent (1% v/v suspension) were added in LP3 tubes (Luckenham) to a solution of the antibody to be captured at a range of concentrtion (1-100 µg/ml). The tubes were incubated for 15 minutes at 20°C and then the cells were deposited by centrifugation and washed once, before resuspending to their original volume. Alternatively, the tubes could be filled with PBS before centrifugation when the deposited red cells could be resuspended for use without the washing step.

The red cells (1% suspension) with captured antibody were then added to titrations of antigen in microtitre plates (using equal volumes of 30 µl) ; the plates were shaken to mix the contents of the wells and the pattern of haemagglutination read after the red cells had sedimented (usually 1 hour).

RESULTS

(1) Capturing monoclonal mouse IgG antibodies for the detection of Chlamydia psittaci antigens – testing different monoclonal antibodies

E6 bovine red cells were coupled with an immunoglobulin-rich preparation of sheep anti-mouse Ig (A366). Equal volumes of 1% coupled red cells were incubated with varying dilutions of different anti-chlamydia monoclonal antibodies (Thornley et al, 1985) (Table 2). The unagglutinated red cells, coated with mouse monoclonal antibodies, were then washed and each suspension used to measure chlamydia antigen in dilutions of a psittacosis complement fixation antigen preparation. The results with five different monoclonal antibodies are set out in Table 2.

The chlamydial antigen could be detected to a dilution of 1/640, which is comparable with the level measurable by the usual RPH (Thornley et al, 1985). Optimal sensitivity in chlamydial antigen detection was obtained after exposure of the antiglobulin-coupled bovine red cells to a 1/500 dilution of the monoclonal antibodies (a drop in sensitivity was seen when the dilution was increased to 1 in 5000). A drop in sensitivity in antigen detection was also noticed with concentrations of the monoclonal antibodies greater that the 1/500. No agglutination (RPH) occurred in the absence of antigen [except for antiglobulin-linked red cells in the presence of a 1/5 dilution of CF2/47] or in the absence of captured antibody. In performance of the test, antiglobulin-linked bovine red cells could be washed six times after capturing the monoclonal antibodies without loss of titre in detection of microbial antigen.

The results given in Table 2 are of special interest because it had previously not been possible to couple the monoclonal antibody, CF6/C5, directly to enzyme-treated sheep red cells by chromic chloride thus precluding its use in the usual RPH reaction. Also the two other monoclonal antibodies, CF4/B2 and CF6/L13, were extremely difficult to couple directly.

(2) Capturing mouse monoclonal antibodies to rotavirus and to Herpes simplex virus on antiglobulin-linked bovine red cells

Two further systems of mouse monoclonal antibodies and their corresponding viral antigens were examined.

8

2(i) Anti-rotavirus

Antiglobulin-linked inagglutinable bovine red cells were prepared and incubated with aliquots of A3M4 – a γ2a anti-rotavirus, monoclonal antibody diluted 1/5, 1/50, 1/500 and 1/1000 of a 500 μg/ml concentration. After washing, the unagglutinated cell suspensions (with captured monoclonal antibodies) were used to measure viral antigen in a control standard infant stool preparation containing rotavirus. All the cell suspensions gave a "Capture RPH" reaction to a dilution of 1/500 of the stool preparation – the same limit of sensitivity as obtained with the usual RPH using the A3M4 monoclonal antibody coupled directly to enzyme-treated sheep red cells (Cranage et al, 1985).

2(ii) Anti-Herpes simplex virus

The results of testing the four monoclonal antibodies on culture preparations of the Type 1 and 2 viruses are shown in Table 3. The optimal dilution of the Type 1 specific monoclonal LP10 was 1/5 while for the Type 2 specific monoclonal antibody (LP4) the optimal dilution for the capture was 1/50.

(3) "Capture RPH" for assay of meningococcal polysaccharides and selective capture of either IgG or IgM monoclonal antibodies

Using a mouse IgG (γ2b) monoclonal antibody (WMA 33) to meningococcal group A polysaccharide and a mouse IgM monoclonal antibody (MB67/A7) to meningococcal B polysaccharide, a capture RPH with anti-mouse Ig-coupled bovine red cells gave very similar results to those obtained with classical RPH (with the monoclonal antibodies linked directly with chromic chloride to enzyme-treated sheep red cells). The optimal dilution of both the anti-A and anti-B monoclonal antibodies was 1/10. Specific detection of both A and B polysaccharide antigen extended to approximately 100 pg/ml. Antigen-excess prozones occurred with concentrations of polysaccharides above 100 ng/ml

The polyclonal sheep anti-mouse Ig reagent (A 366) was not isotype specific, but two other polyclonal anti-mouse Ig reagents (Z706E and Z609C) were specific for IgM and IgG respectively. The IgM monoclonal antibody to meningococcal B polysaccharide was captured and functioned in the "capture RPH" only on bovine red cells coupled with the anti-μ reagent and similarly the IgG monoclonal to meningococcal A polysaccharide functioned only with red cells coupled with the anti-mouse γ-specific reagent. This showed the specificity of the capturing stage. A further illustration, this time in the chlamydia test system, showed that the IgG (CF6/J12 γ2a) mouse monoclonal antibody to the chlamydial group antigens was captured and functional in capture RPH on bovine red cells coupled with the specific anti-mouse γ reagent but not on cells coupled with the specific anti-mouse μ reagent.

(4) Stabilization of the antiglobulin coupled "inagglutinable" bovine red cells used in the capture RPH assay

The antiglobulin-coupled bovine red cells could be stabilized by mild treatment with glutaraldehyde (Cranage et al, 1983, J. Immunol. Methods 64, 7-16) and subsequently freeze-dried with very little loss of activity. This was tested by subsequent capturing the CF6/J12 mouse monoclonal antibody and detecting psittacosis antigen and also by capturing A3M4 mouse monoclonal antibody and detecting rotavirus. Thus freeze-dried antiglobulin-linked bovine red cells could, in theory, be used to "capture" any monoclonal antibody and effect a "capture RPH assay".

Example 3 : Detection of IgE

Red blood cells

Blood was drawn weekly from a single cow and a single sheep into acid-citrate-dextrose (ACD). The red cells were washed and centrifuged (2000 g) five times. All buffy coat was removed.

Antibodies

(i) Mouse monoclonal antibodies (154/102 and 154/117) to human IgE (Alterman et al, Clin. exp. Immunol. 67, 617-625, 1987) were purified from ascitic fluid by 40% saturated ammonium sulphate precipitation.
(ii) Chimaeric mouse/human IgE anti-4-hydroxy-3-iodo-5-nitrophenacetyl (NIP) was used as a model IgE. The chimaeric IgE was prepared as described by Neuberger et al (Nature 31, 268-270, 1985). The IgE was

9

provided as a culture supernatant.

All antibodies for coupling were dialysed exhaustively against 0.9% w/v NaCl.

## NIP-Labelling

NIP-azide (21 mg/ml in DMSO, 80 μl) was conjugated to bovine serum albumin (BSA) (40 mg/ml, 0.5 ml) in bicarbonate buffer, pH 8.8 for 20 hrs at 4°C (Brownstone et al, Immunology 10, 465-479, 1966).

Some of the NIP-BSA (1 mg/ml in NaCl 0.9% w/v) was coupled to chymotrypsin-treated sheep red cells (chtSRBC) by the $CrCl_3$ method. Conjugation of NIP-azide directly gave products unsuitable for use in the assay systems. The chtSRBC were obtained by treating SRBC (5% v/v) with chymotrypsin (Sigma C.4129, final concentration 0.1 mg/ml) by the method of Siddle et al (J. Immunol. Methods 73, 169-176, 1984).

## Coupling of Antibodies to RBC $CrCl_3$

Coupling of antibodies to RBC was as described by Siddle et al (1984) but using a reaction time of only 5-10 min. The monoclonal antibodies were coupled at 0.5 mg/ml. Optimal dilutions of 1% w/v $CrCl_3$ were obtained by checkerboard titration.

## Performance of the red cell IgE antibody capture assay

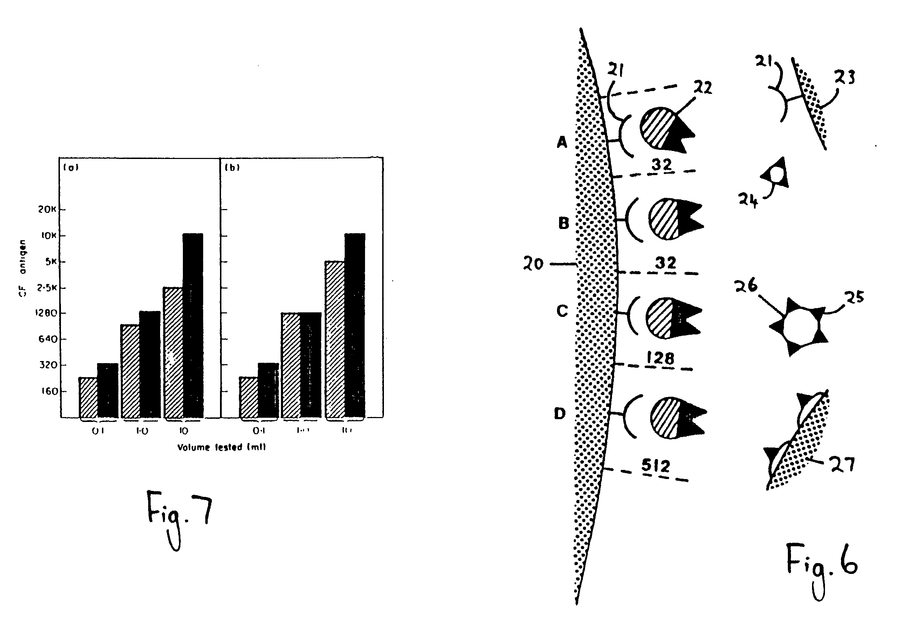

The red cell-capture procedure for capturing and measuring allergen-specific IgE is shown in Figure 6. In the experiments relatively 'inagglutinable' bovine red cells (E6) (20) were coupled with monoclonal anti-IgE (21). The coupled red cells, as a 1% suspension were added to titrations of the culture supernatant containing the chimaeric IgE anti-NIP (22). After washing the still unagglutinated red cells were exposed to BSA-NIP antigen, presented in the different ways shown as A to D in Figure 6.

A : (comparison) The captured IgE was revealed by adding, in the final step, highly agglutinable chtSRBC cells (23) coupled with anti-IgE (21). Haemagglutination was shown to a dilution of 1 in 32 of the culture supernatant.

B : The uptake of NIP-specific chimaeric antibody was also shown to a similar level (i.e. a 1/32 dilution) by presenting the unagglutinated capturing red cells in the last step with BSA-NIP (24) at an optimal dilution (0.2 μg/ml). Again the titre of IgE anti-NIP detected in the culture supernatant was 32.

C : The capture assay was even more sensitive when the NIP determinant (25) was presented conjugated on mouse IgM (25), a larger molecule than BSA (900 kD as opposed to 67 kD). The titre of IgE anti-NIP detected in this assay was 128.

D : The most sensitive detection was achieved in this capture assay when the NIP-antigen in the final step was presented on very agglutinable enzyme-treated sheep red cells (27) (cells coupled with BSA-NIP). The titre of IgE anti-NIP measured in the culture supernatant in this case was 512.

## Example 4 : Detection of Chlamydia psittaci in large volumes of sample

E6 bovine red cells were coupled with an immunoglobulin-rich fraction of a specific anti-mouse IgG designated AX609C as described in Example 2. The antiglobulin-linked bovine red cells were examined both fresh and stabilised with glutaraldehyde. A 1% suspension of coupled red cells was incubated with 200 ng ml$^{-1}$ mouse anti-chlamydia J12 monoclonal ($IgG_{2a}$ isotype) specific for the lipopolysaccharide group antigen (Thornley et al, J. Gen. Microbiol. 131, 7-15, 1985). The cells were centrifuged down and resuspended in PBS.

Psittacosis CF antigen from the Public Health Laboratory Service was examined in a dilution series from 1/640 dilution in PBS azide with 0.1% BSA added. Titrations of the psittacosis antigen were made twice in 100 μl, 1 ml and 10 ml volumes. 100 μl of the suspension in PBS of the bovine red cells coupled with the anti-chlamydia antibody was added to each volume.

After incubation at room temperature either for 30 minutes or overnight the red cells with their affinity-caputured antigen were centrifuged at 1000 g for 15 minutes. The deposited red cells were resuspended in 200 μl of their own supernatant fluid. 60 μl volumes of each tube i.e. two volumes of 0.5% suspension) were transferred to corresponding wells of microtitre plates. The haemagglutination (RPH) end-point was read by naked eye once the red cells had sedimented. The results are shown in Figure 7.

TABLE 1     <u>Preliminary 'blind test' on six serum samples for Mycoplasma pneumoniae-specific IgM using the red cell IgM-antibody capture assay</u>

| | µ-Capture ELISA Test antibody units | Red Cell IgM-antibody capture assay titre | Usual Homogeneous RPH† titre | Mixed-two stage RPH† titre |
|---|---|---|---|---|
| Serum 1 | >33U | >10,000 | 640,000 | 10,000 |
| 2 | >3.3U | 1,000 | 160,000 | 2,500 |
| 3 | 0 | 0* | 640,000 | 10,000 |
| 4 | 0 | 0* | 160,000 | 2,500 |
| 5 | 0 | 0* | 10,000 | 160 |
| 6 | >3.3U | 500 | 640,000 | 2,500 |
| Known Negative | 0 | 0* | 40,000 | 1,000 |
| Known Positive | >33U | >10,000 | 160,000 | 2,500 |

0* = <10

† RPH = Reverse Passive Haemagglutination for IgM in serum. The usual RPH where antibody-linked enzyme-treated sheep red cells are added to dilutions of the ligand is a homogeneous reaction with no washing step. The two-stage test was where antiglobulin (µ-specific)-linked enzyme-treated sheep red cells were added to E6 bovine red cells with 'captured' IgM and which had also been washed. This test was done to correspond to the red cell IgM antibody capture assay.

Table 2 Capturing anti-chlamydia mouse monoclonal antibodies onto bovine red cells and assay of chlamydial antigen by capture RPH

| Antibody captured onto Antiglobulin-coupled E6 bovine red cells (conc) dilution used: | Antigen* used for titration | Antigen dilution 1 in – (Twofold dilution of antigen) | | | | | | | | | | Diluent only |
|---|---|---|---|---|---|---|---|---|---|---|---|---|
| | | 10 | 20 | 40 | 80 | 160 | 320 | 640 | 1280 | 2560 | 5120 | |
| CF6/J12 (0.66 mg/ml) 1/5 | Psittacosis CF. antigen | ++ | ++ | ++ | ++ | ++ | W | - | - | - | -- | - |
| 1/50 | | ++ | ++ | ++ | ++ | ++ | ++ | W | - | - | - | - |
| IgG2a 1/500 | | ++ | ++ | ++ | ++ | ++ | ++ | ++ | W | - | - | - |
| CF2/47 (0.45 mg/ml) 1/5 | " | ++ | ++ | ++ | ++ | ++ | ++ | ++ | ++ | ++ | ++ | ++ |
| 1/50 | | ++ | ++ | ++ | ++ | ++ | ++ | + | W | - | - | - |
| IgG3 1/500 | | ++ | ++ | ++ | ++ | ++ | ++ | + | W | - | - | - |
| CF6/L13 (0.8 mg/ml) 1/5 | " | ++ | ++ | ++ | + | W | - | - | - | - | - | - |
| 1/50 | | ++ | ++ | ++ | ++ | ++ | ++ | W | - | - | - | - |
| IgG2a 1/500 | | ++ | ++ | ++ | ++ | ++ | ++ | ++ | W | - | - | - |
| CF4/132 (0.28 mg/ml) 1/5 | " | ++ | ++ | ++ | + | W | - | - | - | - | - | - |
| 1/50 | | ++ | ++ | ++ | ++ | + | W | - | - | - | - | - |
| IgG3 1/500 | | ++ | ++ | ++ | ++ | ++ | ++ | W | - | - | - | - |
| CF6/L5 (0.86 mg/ml) 1/5 | ". | ++ | ++ | ++ | ++ | ++ | + | W | - | - | - | - |
| 1/50 | | ++ | ++ | ++ | ++ | ++ | ++ | + | - | - | - | - |
| IgG2a 1/500 | | ++ | ++ | ++ | ++ | ++ | ++ | ++ | W | - | - | - |
| diluent only | " | - | - | - | - | - | - | - | - | - | - | - |

* Antigen = CF antigen 8202/2
++ + w = degrees of haemagglutination
- = no haemagglutination

12

## Table 3 Detection of Herpes simplex antigen

| Using Monoclonal antibodies:- | Culture preparation of Herpes simplex virus. Levels of detection:- | |
| --- | --- | --- |
| | Type 1* | Type 2* |
| AP7 + LP2 | 20 | 40 |
| LP10 | 40 | 0 |
| LP4 | 0 | 320 |

Level of detection - reciprocal of dilution

* Type 1 Strain HFEM

  Type 2 Strain HVD

## Claims

1. A method of assaying a sample for (a) an antibody specific for a predetermined antigen having multiple epitopes or (b) a predetermined antigen having multiple epitopes, characterised by :

(i) contacting inagglutinable bovine red blood cells to which an immunoglobulin binding agent is attached with the sample in the case of assay for the said antibody (a) or with antibody specific for the said antigen (b) in the case of assay for the said antigen (b) ;

(ii) contacting the thus-treated red cells with antigen for which the said antibody (a) is specific in the case of assay for the said antibody (a) or with the sample in the case of assay for the said antigen (b) ; and

(iii) determining whether haemagglutination occurs.

2. A method according to claim 1, wherein the immunoglobulin binding agent is an antiglobulin.

3. A method according to claim 2, wherein the immunoglobulin binding agent is anti-γ antibody, anti-μ antibody or anti-ε antibody.

4. A method according to any one of the preceding claims, wherein the test sample is human serum.

5. A method according to any one of the preceding claims, wherein an antibody is assayed for by adding a suspension of the red cells to which the immunoglobulin binding agent is attached to the test sample ; incubating the test sample with the added red cells ; removing, washing and resuspending in buffer solution the thus incubated red cells ; adding antigen for which the antibody under assay is specific ; and determining whether haemagglutination occurs.

6. A method according to any of claims 1 to 4, wherein an antigen is assayed for by bringing antibody specific for the antigen into contact with the red cells to which the immunoglobulin binding agent is attached ; adding the test sample and determining whether haemagglutination occurs.

7. A method according to claim 6, wherein the said antibody specific for the antigen is a monoclonal antibody.

8. A method according to any one of the preceding claims, wherein the said predetermined antigen having multiple epitopes is a microbial pathogen.

9. A test kit suitable for use in an assay for (a) an antibody specific for a predetermined antigen having

13

multiple epitopes or (b) a predetermined antigen having multiple epitopes, characterised by comprising :

(i) inagglutinable bovine red blood cells to which an immunoglobulin-binding agent is attached ; and

(ii) in the case of assay for the said antibody (a), antigen for which the said antibody (a) is specific or, in the case of assay for the said antigen (b), antibody specific for the antigen (b).

10. A kit according to claim 9, wherein the immunoglobulin-binding agent is an antiglobulin.

## Ansprüche

1. Verfahren zum Untersuchen einer Probe auf (a) einen für ein vorgegebenes Antigen mit mehrfachen Epitopen spezifischen Antikörper oder (b) ein vorgegebenes Antigen mit mehrfachen Epitopen, dadurch gekennzeichnet, daß :

(i) nicht-agglutinierbare rote Rinderblutzellen, an die ein Immunoglobulinbindemittel gebunden ist, mit der Probe im Falle der Untersuchung auf den Antikörper (a) oder mit für das Antigen (b) spezifischem Antikörper im Falle der Untersuchung auf das Antigen (b) in Berührung gebracht werden ;

(ii) die derart behandelten roten Zellen mit Antigen, für das der Antikörper (a) spezifisch ist, im Falle der Untersuchung auf den Antikörper (a) oder mit der Probe im Falle der Untersuchung auf das Antigen (b) in Berührung gebracht werden ; und

(iii) festgestellt wird, ob Hämagglutination auftritt.

2. Verfahren nach Anspruch 1, worin das Immunoglobulinbindemittel ein Antiglobulin ist.

3. Verfahren nach Anspruch 2, worin das Immunoglobulinbindemittel ein Anti-γ-Antikörper, ein Anti-μ-Antikörper oder ein Anti-ε-Antikörper ist.

4. Verfahren nach einem der vorhergehenden Ansprüche, worin die Testprobe menschliches Serum ist.

5. Verfahren nach einem der vorhergehenden Ansprüche, worin dadurch auf einen Antikörper untersucht wird, daß eine Suspension der roten Zellen, an die das Immunoglobulinbindemittel gebunden ist, der Testprobe zugegeben wird ; die Testprobe mit den zugegebenen roten Zellen inkubiert wird ; die derart inkubierten roten Zellen entfernt, gewaschen und in Pufferlösung resuspendiert werden ; Antigen, für das der zu bestimmende Antikörper spezifisch ist, zugegeben wird ; und festgestellt wird, ob Hämagglutination auftritt.

6. Verfahren nach einem der Ansprüche 1 bis 4, worin dadurch auf ein Antigen untersucht wird, daß für das Antigen spezifischer Antikörper mit den roten Zellen, an die das Immunoglobulinbindemittel gebunden ist, in Berührung gebracht wird ; die Testprobe zugegeben und festgestellt wird, ob Hämagglutination auftritt.

7. Verfahren nach Anspruch 6, worin der für das Antigen spezifische Antikörper ein monoklonaler Antikörper ist.

8. Verfahren nach einem der vorhergehenden Ansprüchen, worin das vorgegebene Antigen mit mehrfachen Epitopen ein mikrobielles Pathogen ist.

9. Testausrüstung, geeignet zur Verwendung in einer Untersuchung auf (a) einen für ein vorgegebenes Antigen mit mehrfachen Epitopen spezifischen Antikörper oder (b) ein vorgegebenes Antigen mit mehrfachen Epitopen, dadurch gekennzeichnet, daß sie

(i) nicht-agglutinierbare rote Rinderblutzellen, an die ein Immunoglobulinbindemittel gebunden ist ; und

(ii) im Falle der Untersuchung auf den Antikörper (a) Antigen, für das der Antikörper (a) spezifisch ist, oder im Falle der Untersuchung auf das Antigen (b) für das Antigen (b) spezifischen Antikörper umfaßt.

10. Ausrüstung nach Anspruch 9, worin das Immunoglobulinbindemittel ein Antiglobulin ist.

## Revendications

1. Procédé pour doser dans un échantillon (a) un anticorps spécifique d'un antigène déterminé au préalable ayant des épitopes multiples ou (b) un antigène déterminé au préalable ayant des épitopes multiples, lequel procédé comprend :

(i) la mise en contact de globules rouges de boeuf non agglutinables auxquels est attaché un agent de fixation d'une immunoglobuline avec l'échantillon dans le cas du dosage de l'anticorps (a) ou avec un anticorps spécifique de l'antigène (b) dans le cas du dosage de l'antigène (b) ;

(ii) la mise en contact des globules rouges ainsi traités avec un antigène pour lequel l'anticorps (a) est spécifique dans le cas du dosage de l'anticorps (a) ou avec l'échantillon dans le cas du dosage de l'antigène (b), et

(iii) la détermination s'il y a ou non hémagglutination.

2. Procédé suivant la revendication 1, dans lequel l'agent de fixation d'une immunoglobuline est une antiglobuline.

3. Procédé suivant la revendication 2, dans lequel l'agent de fixation d'une immunoglobuline est un anticorps anti-γ, un anticorps anti-μ ou un anticorps anti-ε.

4. Procédé suivant l'une quelconque des revendications précédentes, dans lequel l'échantillon à examiner est du sérum humain.

5. Procédé suivant l'une quelconque des revendications précédentes, dans lequel un anticorps est dosé par addition d'une suspension des globules rouges auxquels est attaché l'agent de fixation d'une immunoglobuline à l'échantillon à examiner ; par incubation de l'échantillon à examiner avec les globules rouges ajoutés; par collecte, lavage et remise en suspension dans de la solution tampon des globules rouges ainsi incubés ; par addition d'un antigène pour lequel l'anticorps soumis au dosage est spécifique ; et par détermination s'il y a ou non hémagglutination.

6. Procédé suivant l'une quelconque des revendications 1 à 4, dans lequel l'antigène est dosé en mettant l'anticorps spécifique pour l'antigène en contact avec les globules rouges auxquels est attaché l'agent de fixation d'une immunoglobuline ; en ajoutant l'échantillon à examiner et en déterminant s'il y a ou non hémagglutination.

7. Procédé suivant la revendication 6, dans lequel l'anticorps spécifique pour l'antigène est un anticorps monoclonal.

8. Procédé suivant l'une quelconque des revendications précédentes, dans lequel l'antigène déterminé au préalable ayant des épitopes multiples est un pathogène microbien.

9. Trousse de test se prêtant à l'utilisation dans un dosage pour rechercher (a) un anticorps spécifique pour un antigène déterminé au préalable ayant des épitopes multiples ou (b) un antigène déterminé au préalable ayant des épitopes multiples, caractérisée en ce qu'elle comprend :

(i) des globules rouges de boeuf non agglutinables auxquels est attaché un agent de fixation d'une immunoglobuline, et

(ii) dans le cas du dosage de l'anticorps (a), un antigène pour lequel l'anticorps (a) est spécifique ou dans le cas du dosage de l'antigène (b), un anticorps spécifique pour l'antigène (b).

10. Trousse suivant la revendication 9, dans laquelle l'agent de fixation d'une immunoglobuline est une antiglobuline.

# Fig.1.

# Fig.2.

ABSORBANCE READ

SUBSTRATE ADDED

READ FOR
HAEMAGGLUTINATION

WELL OF MICROTITRE PLATE

BOVINE RED CELL

# Fig.3.

## Fig.4.

## Fig.5.

Fig. 6

Fig. 7